Europäisches Patentamt

(19) European Patent Office

Office européen des brevets

(11) Publication number: **0 002 068**
**B1**

(12) **EUROPEAN PATENT SPECIFICATION**

(45) Date of publication of patent specification: **02.12.81**

(51) Int. Cl.³: **A 61 N 1/04, A 61 F 11/04**

(21) Application number: **78101421.2**

(22) Date of filing: **21.11.78**

(54) Electrode for implantation into cochlea.

(30) Priority: **22.11.77 DK 5167/77**

(43) Date of publication of application:
**30.05.79 Bulletin 79/11**

(45) Publication of the grant of the European patent:
**02.12.81 Bulletin 81/48**

(84) Designated Contracting States:
**BE CH DE FR GB LU NL SE**

(56) References cited:
**US - A - 3 752 939**
**US - A - 3 890 977**

**MEDICAL & BIOLOGICAL ENGINEERING, vol. 12, no. 6, (November 1974) Stevenage GB M. SONN et al. "A prototype flexible microelectrode array for implant-prosthesis applications", pages 778—790.**

(73) Proprietor: **Hansen, Carl Christian**
**Sadolinsgade 114 B**
**DK-5000 Odense (DK)**

(73) Proprietor: **Lauridsen, Ole Mork**
**Kärvej 13**
**DK-3520 Farum (DK)**

(72) Inventor: **Hansen, Carl Christian**
**Sadolinsgade 114 B**
**DK-5000 Odense (DK)**
Inventor: **Lauridsen, Ole Mork**
**Kärvej 13**
**DK-3520 Farum (DK)**

(74) Representative: **Fleck, Thomas, Dr.**
**Geffckenstrasse 6**
**D-2000 Hamburg 20 (DE)**

Electrode for implantation into cochlea

The present invention relates to an implantable flexible electrode for implantation into the cochlea and disposed to establish electrical communication with the auditory nerves of the human ear comprising an elongate foil-like support member of a flexible electrically insulating material and a pattern of electrically conductive paths of a material inert to body fluids and tissue applied to the surface of said support member and in which each of said paths extends along the surface and terminates in an enlarged exposed contact area as described by Martin Sonn and Wolfgang Feist in an article entitled "A prototype flexible microelectrode array for implant-prosthesis applications" in Medical and Biological Engineering, November 1974, pages 778—790.

The prior art electrodes are disposed to be inserted through an opening into a patient's cochlea, whose internal ear is defective. In order to establish communication substantially in the area of the cochlea which is relevant to the intelligibility of speech the electrodes are provided with a length so that they can reach through approximately two of the cochlea's two and a half turns. It has appeared, however, that the purpose has not been attained because, although it has certainly been possible to bring the patient to such a condition that she or he could interpret electrical signals supplied through the electrode as being sound, the patient has not been able to interpret the signals as intelligible speech.

The present invention is based on the opinion that this phenomenon is partly due to the fact that the prior art electrodes, during the insertion, had to be guided in the cochlea by the aid of the walls of the cochlea. During this insertion damage occured to these walls. This resulted in the fact that the electrode, even if the insertion was tolerably successful, would not be able to position itself in the cochlea in such a manner that the supplied signals were conducted selectively to the auditory nerves for which they were destined for evoking in the brain impressions which could cause it to perceive the electrical signals as communicating, intelligible speech.

The present invention aims at avoiding the drawbacks associated with the prior art electrodes. Accordingly, the present invention provides an electrode in which the part to be accommodated in the cochlea has an eigencurvature in the longitudinal direction of the support member which corresponds to the curvature of the cochlea wall containing the nerve endings in the particular turn of the cochlea into which it is to be inserted. The electrode is further provided with means for releasably prestressing said part of the electrode to attain a temporary curvature corresponding to a datum line in the middle of said turn of the cochlea, the prestressing means being capable of being released when the curved electrode part is secured in place in the cochlea turn and thus permitting said electrode part to return to its eigencurvature condition establishing optimum engagement between nerve endings and exposed electrode contact areas.

In this manner not only the possibility of a nearly contactless insertion of the electrode into the cochlea is achieved so that its walls are not further damaged, but additionally, the electrode, after being inserted into the desired turn of the cochlea, can be given such a required curvature so that the electrode comes into contact with the internal wall of the cochlea duct which contains the nerve endings of the auditory nerves. The electrode is designed to assume a constant position relative to the auditory nerves throughout the various sections of the cochlea so that the enlarged exposed contact areas of the electrode, through which the electrical signals have to be transmitted to the proper auditory nerves, can obtain a constant and an optimum position relative to the auditory nerves.

At this junction it should be noted that there is no need for adapting the electrode to individual persons since the size of the cochlea is almost constant all through the patient's life.

In US—PS 3,752,939 there is disclosed a prosthetic device for the deaf, in which the supply lines or conductors, when the electrode has been implanted and secured in place, extend parallel to the length of the basilar membrane and in close proximity thereto. However, the conductors of all the configurations disclosed in said specification are not engaging or touching the basilar membrane so that the electrical field generated between the conductors is transmitted through the conductive cochlea fluid to the nerve endings in the basilar membrane. Hence, an optimal engagement between the nerve endings in the basilar membrane and the conductors is clearly not intended according to said specification.

In the present invention a different concept has been followed, viz. the creation of an implantable electrode which during implantation is forced to assume a temporary condition of curvature to facilitate insertion into the middle of the particular turn of the cochlea and subsequently permitted to assume a final or permanent condition of curvature in which the exposed contact areas individually engage nerve endings in the basilar membrane to optimize the electrode's capability of stimulation.

The transfer from the temporary curvature to the permanent one is done by mechanical means. In US—PS 3,890,977 there is disclosed kinetic memory electrodes, catheters or cannulae which are formed, either wholly or in part, of a material exhibiting mechanical memory, that is triggered by heat. The teaching here is, however, that the electrodes have to be provided with an individual or special member of a material exhibiting mechanical memory, and as suitable materials are mentioned various titanium allows. The material may be present as a rod for example. In contrast to this the present invention utilizes the inherent resiliency of the foil-like support member.

Said prestressing means may comprise wax having a melting or softening point a few degrees centigrade below the body temperature, or a material, such as dried mucus, which is soluble or swellable in the fluid contained in the cochlea, said material being applied on the electrode surface when in a preshaped condition corresponding to said temporary curvature and subsequently dried to establish said prestressed condition.

The advantage of this embodiment is that the electrode, when inserted and secured in place in the cochlea turn and after the release of the prestressing means will be in a mechanically stressless condition so that it is capable of assuming those delicate adjustments of the electrode normally made during operation for insertion of the electrode, for a long period without a tendency to change. This then permits a quick and reliable operation, and the probability of obtaining a uniform communication over various frequency ranges along the cochlea becomes larger. Finally, there is no need for a specific material exhibiting mechanical memory to be incorporated.

The term wax should not be interpreted in a narrow sense but also to cover other materials than organic waxes which have a similar softening temperature and are inert to body tissue and fluids in the surroundings mentioned.

In an alternative embodiment of the invention the prestressing means comprises tearable mechanical connections, such as spot welds, between a longitudinal edge of said support member and opposing electrode contact areas, said mechanical connections being provided when said electrode part, to establish said prestressed condition, is folded laterally around a resilient pressure tube or hose containing a fluid and shaped to conform to said temporary curvature. When the pressure in said tube or hose is increased the tube or hose expands and thereby releases said connections.

Spot welds have been mentioned above, which provide the smallest area of welds, but a continuous weld could also be appropriate.

In still another embodiment of the invention the prestressing means comprises a cord provided within or on the insulating support member. Pulling or releasing the cord adjusts or controls continuously the curvature of the electrode part to be inserted into the cochlea in order to provide said temporary curvature of the electrode part. This facility permits special adaptations to abnormal conditions which may be encountered during surgery, e.g. in connection with cranial fractures or deformities which require exceptional arrangements.

In yet another embodiment of the invention the prestressing means comprises a curved tube having an oval cross-section and containing a pressure medium rendering it operable like a Bourdon tube in adjusting the condition of curvature to define said temporary curvature. This arrangement implies similar advantages as mentioned above.

The invention will be further described in details below with reference to the accompanying drawings, in which:

Figure 1 is a sectional view through the centre line in a single turn of a space in the cochlea during an operation for insertion of an electrode according to the invention,

Figure 2 is a planar view of an embodiment of an electrode according to the invention in a flat condition,

Figure 3 is a schematic perspective view of a former onto which an electrode is applied for setting a temporary condition of curvature according to the invention,

Figure 4 is a schematic perspective view of another former, around which an electrode according to the invention can be placed while it is given a temporary condition of curvature, and

Figures 5 to 7 are cross-sectional views of further embodiments of an electrode according to the invention.

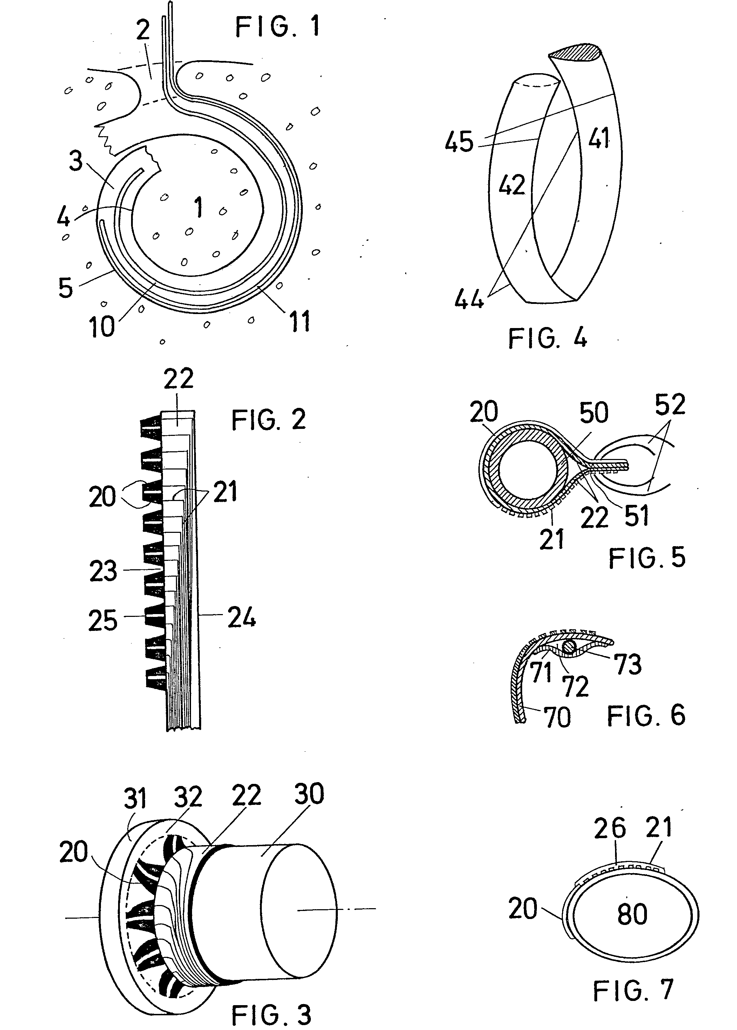

In Figure 1, reference numeral 1 indicates a section in a cranium, in which an opening 2 has been provided, which opening leads to a turn of the cochlea. This turn contains three spaces and normally one would introduce the electrode into the lower space 3. In the wall between said space and the intermediate space is the basilar membrane and the auditory nerves extend from the brain to the core of the cochlea and further to the field above the lower space. The lower space 3 has an internal wall 4 and an external wall 5 and forms a helix. It is now possible to introduce the electrode according to the present invention through the opening 2. During the insertion the electrode has a curvature, which corresponds to a centre or datum line between the internal wall 4 and the external wall 5. This curvature is called "the temporary condition of curvature" 10. An electrode assuming the temporary condition of curvature 10 is illustrated in Figure 1 immediately after its introduction. However, this position is not stable, as the electrode is floating, so to speak, in the lymph fluid in the lower space. Therefore, a fixation of the electrode relative to the internal or external wall is important for enabling the current field between the nerve electrodes over the entire length of the inserted electrode part to contact accurately and uniformly the desired zones having nerve endings. Accordingly, the electrode of the present invention has a permanent condition of curvature 11, which may have either a smaller radius of curvature or, as illustrated in Figure 1, a larger radius of curvature than the temporary one. Hence, the electrode will either nestle against the internal wall 4 or against the external wall 5 as shown in Figure 1. Thus, the electrode of the present invention has been given the combination of properties required for communication directly to the auditory nerves: i.e. it has been so shaped that it can be introduced without damaging the walls of cochlea and in such manner that it can be fixed relative to the nerve endings uniformly over the entire length of the electrode part introduced into the cochlea. It can be said that apparently there is a discrepancy between the requirement of the two properties mentioned, but the discrepancy has been brought to a conclusion by the inventive realization of the necessity of an electrode capable of assuming at least two conditions of curvature, a temporary one 10 and a permanent one 11.

Consequently, the electrode according to the invention must include means for changing or enabling the change of curvature from the temporary condition of curvature 10 to a permanent one 11.

When describing the present invention the term "natural condition of curvature" is employed. This means that the curvature which an electrode will assume if it is not forced to assume another curvature, as a result of an external stress imposed by a band, for example. Such a band can be disposed to straighten the electrode or even to give it a curvature of opposite direction. This can be done by temporarily giving the electrode internal stresses, which changes temporarily the natural condition of curvature. Instead of this term one could use the term "eigencurvature". In carrying out the invention the electrode part to be inserted into the cochlea is provided with means for releasably prestressing said electrode part to assume a temporary condition of curvature corresponding to the above mentioned datum line. When this means is released the electrode part will revert to its natural condition and again assume its eigencurvature.

In Figure 2, there is illustrated that part of the electrode according to the invention which carries the nerve electrodes 20 in the shape of enlarged exposed contact areas. These areas are to be positioned in the cochlea. The remaining part of the electrode carries the connecting or supply lines 21 from the part illustrated to a signal generating apparatus (not shown). An electrode in this path of connections will usually have a base and a socket associated therewith permitting a separation between the patient and the signal apparatus. As neither the base, nor the socket nor the signal apparatus relate to the electrode according to the invention in another way than as necessary accessories when the patient under operation is going to use the electrode, these parts have not been illustrated in the drawing and can be of conventional types. The nerve electrodes 20 are arranged in pairs in a well-known manner, and a conductor or supply line 21 leads to each nerve electrode. The nerve electrodes 20 as well as the conductor lines 21 are secured to an insulating support member 22 of a plastic foil having a suitable thickness, suitable properties of resilience and the property so that the foil is inert to body tissue and fluids, which also is required for the material of the nerve electrode and the conductor line. Conductor lines and electrodes can be applied on the foil by the aid of any known method by thin film technique. The outline of the electrode is stamped out, cut out or punched out also by employing well-known techniques. Separating each pair of electrodes 20 there are contoured V-shaped notches 23 which extend into one of the longitudinal edges. These notches serve to permit the required generally toroidal shape, which the electrode must have in its temporary position as well as in its permanent position. Appropriately, the conductor lines 21 are covered by an insulating layer delimiting an external current field to the immediate proximity of the nerve electrodes 20.

Figure 3 illustrates a former or mandrel comprising two cylindrical members 30 and 31 and therebetween a former member 32 of generally toroidal shape. The former or mandrel is disposed to be heated to a temperature above the softening temperature of the insulating support member 22. The former member 32 provides the same shape as the wall in the cochlea at the position of permanent placement of the electrode. By placing the electrode so that its insulating suppport member 22 and nerve electrodes 20 are contacting the former member 32 and subsequently cooling the former or mandrel from a temperature above the softening temperature to below the solidifying temperature, the electrode is caused to assume its natural condition of curvature or eigencurvature. It is to be understood from Figure 3 that the nerve electrodes and the conductor lines are facing the former or mandrel whereas the insulating support member is illustrated as being transparent. Thus, the electrode is seen from the back of the printed circuit connections.

Subsequently, an electrode fashioned as mentioned above is wrapped around another shaping member, which is illustrated in Figure 4. This member is shaped like a flat ring, which is cut open and wrung a little out of plane so that its curvature corresponds to the temporary condition of curvature 10 illustrated in Figure 1. Referring to Figure 4, the cross-section of the ring is sharp-edged on the left-hand side 44 and round-edged on the right-hand side 45. The electrode is arranged on the "ring" with its back facing the ring and the nerve electrodes or contact areas 20 folded into the inside 41 of the ring whereas the conductor side is on the outside 42 of the ring. The circumference of the cross-section of the ring is a little shorter than the distance between the edge 24 of the insulating support member and the tips 25 of the nerve electrodes 20. This has the effect that the electrode tips 25 will contact the edge 24 at the left-hand, sharp edge 44. It is at those contacts that the prestressing means is established in some of the embodiments of the invention.

As it appears from Figure 4, the end of the ring, which is visible, has a slightly larger circumference than the other end, which is indicated partly in a dotted line. This design results in that the electrode can be pulled off the ring like a stocking after the prestressing means has been provided.

In some of the embodiments, the prestressing means are inserted between the edge 24 and the electrode tips 25 and in some other embodiments, in which use is made of wax or mucus, the prestressing means are best established by dipping or by spraying the material while an auxiliary tool keeps the electrode members in position on the ring as discussed above with reference to Figure 4.

An embodiment, in which the prestressing means is tearable, is illustrated in Figure 5. In this embodiment, the electrode is folded around an elongate hose 50, which is accessible from the outside, instead of around the ring. One end of the hose 50 is given a curvature similar to that of the ring. It is then possible by means of a spot weld tool 52 to provide tearable spot welds 51 between the nerve electrode side of the electrode and its conductor side and fairly close to the hose 50. This measure enables the hose 50 to tear up the spot welds by letting the hose expand resiliently by increasing the pressure in the interior of the hose.

An embodiment of an electrode according to the invention can be given a continuously varying curvature if it is designed as indicated in Figure 6. Figure 6 is a cross-section of such an electrode in a direction orthogonal to its longitudinal axis. In this Figure the thickness of the insulating support member 70 is, for the sake of clarity, shown very exaggerated. The section is illustrated when having its permanent shape. At the back of the printed circuit is attached a pocket 71 and in the cavity 72 of the pocket is inserted a cord 73, one end of which is secured to the tip of the electrode. When the cord is tightened by pulling its opposite free end, the condition of curvature of the electrode can be increased in order to establish an arbitrary, required temporary condition of curvature. Having inserted such a curved electrode into the cochlea, the pull of the cord is loosened whereby the electrode assumes its permanent curvature.

In Figure 7, there is illustrated a cross-section of an electrode shaped as a tube having an oval cross-section. The outer surface of this section carries nerve electrodes 20 and conductor lines 21 which are covered by an insulating layer 26. The electrode can by means of a thin film technique be provided on a thin foil, which subsequently is formed into a tube having a longitudinal seam. The tube can be arranged on a former or a mandrel of similar appearance as that illustrated in Figure 4, but the sides 45 being rounded to the right-hand side as well as to the left-hand side. By heating to a temperature above the softening temperature of the foil and subsequently cooling to a temperature below the hardening temperature, the tube 80 is given its natural condition of curvature 11. A temporary change of the pressure within the tube 80 will give the tube its temporary condition of curvature 10 for insertion into the cochlea. This embodiment of the electrode according to the invention is particularly advantageous in that it is subjected to only negligible mechanical influences when being inserted into the cochlea so that the risk of rupturing the conductor lines 21 is reduced.

The invention may be embodied in other specific forms without departing from the essential characteristics thereof. The present embodiments are therefore to be considered in all respects as illustrative and not restrictive.

## Claims

1. An implantable flexible electrode for implantation into the cochlea and disposed to establish electrical communication with the auditory nerves of the human ear comprising an elongate foil-like support member of a flexible electrically insulating material and a pattern of electrically conductive paths of a material inert to body fluids and tissue applied to the surface of said support member, each of said paths extending along the surface and terminating in an enlarged exposed contact area, characterized in that the part of the electrode to be accommodated in the cochlea has an eigencurvature in the longitudinal direction of the support member which corresponds to the curvature of the cochlea wall containing the nerve endings in the particular turn of the cochlea into which it is to be inserted and in that means are provided for releasably prestressing said part of the electrode to attain a temporary curvature corresponding to a datum line in the middle of said turn of the cochlea, the prestressing means being capable of being released when the curved electrode part is secured in place in the cochlea turn and thus permitting said electrode part to return to its eigencurvature condition establishing optimum engagement between nerve endings and exposed electrode contact areas.

2. An implantable electrode as claimed in claim 1, wherein said prestressing means comprises wax having a melting or softening point a few degrees centigrade below the body temperature, or a material, such as a dried mucus, which is soluble or swellable in the fluid contained in the cochlea, said material being applied on the electrode surface when in a preshaped condition corresponding to said temporary curvature and subsequently dried to establish said prestressed condition.

3. An implantable electrode as claimed in claim 1, wherein said prestressing means comprises tearable mechanical connections, such as spot welds, between a longitudinal edge of said support member and opposing electrode contact areas, said mechanical connections being provided when said electrode part, to establish said prestressed condition, is folded laterally around a resilient pressure tube or hose containing a fluid and shaped to conform to said temporary curvature, the pressure in said tube or hose being adapted to be increased briefly to expand said tube or hose and thereby release said connections.

4. An implantable electrode as claimed in claim 1, wherein said prestressing means comprises a cord provided within or on the insulating support member, pulling or releasing of said cord adjusting or controlling continuously the curvature of said electrode part to be inserted into the cochlea in order to provide said temporary curvature of said electrode part.

5. An implantable electrode as claimed in claim 1, wherein said prestressing means comprises a curved tube having an oval cross-section and containing a pressure medium rendering it operable like a Bourdon tube in adjusting the condition of curvature to define said temporary curvature.

## Patentansprüche

1. Flexible Elektrode zur Implantierung in die Cochlea bzw. Schnecke und zum Herstellen einer elektrischen Verbindung zu den akustischen Nerven im menschlichen Ohr mit einem länglichen folienartigen Trageteil eines flexiblen elektrischen Isolationsmaterials und einem Muster elektrischer Leitungswege aus einem für Körperflüssigkeit und -Gewebe inerten Material, das an der Oberfläche des Trageteils angebracht ist, wobei sich jeder Leitungsweg entlang dieser Oberfläche erstreckt und in einem vergrößerten freiliegenden Kontaktbereich endet, dadurch gekennzeichnet, daß der in der Cochlea aufzunehmende Elektrodenteil eine Eigenkrümmung in Längsrichtung des Trageteils besitzt, die der Krümmung der Cochleawand entspricht, die die Nervenenden in einer besonderen Windung der Cochlea enthält, in welche es eingeführt werden soll, und daß Mittel vorgesehen sind, die diesen Elektrodenteil wiederlösbar vorspannen, um eine temporäre Krümmung zu erreichen, die einer Grundlinie in der Mitte der genannten Windung der Cochlea entspricht, wobei das Vorspannungsmittel gelöst werden kann, wenn der gebogene Elektrodenteil an seinem Platz in der Cochlea-Windung befestigt wird, wodurch dem Elektrodenteil ermöglicht wird, seinen Eigenkrümmungs-Zustand wieder anzunehmen und eine optimale Verbindung zwischen Nervenenden und freigelegten Elektroden-Kontaktbereichen herzustellen.

2. Flexible Elektrode nach Anspruch 1, dadurch gekennzeichnet, daß das Vorspannungsmittel Wachs umfaßt, dessen Schmelz- oder Erweichungspunkt einige Grad Celcius unterhalb der Körpertemperatur liegt oder, daß das Material z.B. getrockneter Schleim ist, der löslich oder in der in der Cochlea enthaltenen Flüssigkeit anschwellbar ist, wobei das Material auf die Elektrodenoberfläche aufgetragen wird, wenn sie in dem vorgeformten Zustand ist, der der temporären Krümmung entspricht und anschließend getrocknet wird, um den vorgespannten Zustand herzustellen.

3. Flexible Elektrode nach Anspruch 1, dadurch gekennzeichnet, daß das Vorspannungsmittel zerreißbare mechanische Verbindungen, z.B. Schweißpunkte, zwischen der Längskante des Trageteils und den sich gegenüberliegenden Elektrodenkontakt-Bereichen umfaßt, und daß die mechanischen Verbindungen vorgesehen werden, wenn der Elektrodenteil, um den vorgespannten Zustand herzustellen, seitlich um ein/einen nachgiebiges Druckrohr oder -Schlauch herumgewickelt wird, welches bzw. welcher ein Strömungsmittel enthält, und derart geformt ist, daß er der temporären Krümmung entspricht, wobei der Druck im genannten Rohr oder Schlauch angepaßt ist, um kurz erhöht werden zu können, um das Rohr oder den Schlauch zu expandieren und dadurch die Verbindungen zu lösen.

4. Flexible Elektrode nach Anspruch 1, dadurch gekennzeichnet, daß das Vorspannungsmittel einen Faden umfaßt, der innerhalb oder auf dem isolierenden Trageteil vorgesehen ist, und daß ein Ziehen oder Lösen des Fadens eine kontinuierliche Einstellung oder Steuerung der Krümmung des in die Cochlea einzusetzenden Elektrodenteils, umdie temporäre Krümmung des Elektrodenteils vorzusehen.

5. Flexible Elektrode nach Anspruch 1, dadurch gekennzeichnet, daß das Vorspannungsmittel ein abgebogenes Rohr umfaßt, das einen ovalen Querschnitt besitzt, und ein Druckmedium umfaßt, daß es wie ein Bourdonrohr beim Einstellen des Krümmungszustandes zur Herstellung der temporären Krümmung betätigt werden kann.

## Revendications

1. Electrode flexible implantable, à implanter dans la cochlée, et disposée pour établir une communication électrique avec les nerfs auditifs de l'oreille d'un être humain, comprenant un élément de support allongé analogue à une feuille en une matière flexible électriquement isolante et un modèle de voies électriquement conductrices en une matière inerte vis-à-vis des fluides corporels et des tissus, qui est appliqué à la surface de l'élément de support, chacune des voies s'étendant le long de la surface et se terminant en une zone de contact à nu, agrandie, caractérisée en ce que la partie de l'électrode à loger dans la cochlée présente une courbure propre dans la direction longitudinale de l'élément de support qui correspond à la courbure de la paroi de la cochlée contenant les terminaisons nerveuses dans l'enroulement particulier de la cochlée dans lequel elle doit être introduite et en ce qu'un moyen est prévu pour tendre préalablement de manière libérable ladite partie de l'électrode pour atteindre une courbure temporaire correspondant à une ligne de repère au milieu dudit enroulement de la cochlée, le moyen de mise sous tension préalable étant capable d'être relâché lorsque la partie d'électrode courbée est fixée en place dans l'enroulement de la cochlée et permettant ainsi à la partie d'électrode de retourner à son état de courbure propre qui établit un engagement optimum entre les terminaisons nerveuses et les zones de contact à nu de l'électrode.

2. Electrode implantable suivant la revendication 1, caractérisée en ce que le moyen de mise sous tension préalable comprend de la cire présentant un point de fusion ou de ramollissement de quelques degrés centigrades en dessous de la température du corps, ou une matière, telle que du mucus séché, qui est soluble ou capable de gonfler dans le fluide contenu dans la cochlée, cette matière étant appliquée sur la surface d'électrode lorsqu'elle est dans un état de façonnage préalable correspondant à la courbure temporaire, et étant ensuite séchée pour établir l'état de tension préalable.

3. Electrode implantable suivant la revendication 1, caractérisée en ce que le moyen de mise sous tension préalable comprend des liaisons mécaniques déchirables, telles que des soudures par points, entre un bord longitudinal de l'élément de support et les zones de contact opposées de l'électrode, ces liaisons mécaniques étant réalisées lorsque la partie d'électrode est, pour établir l'étant de tension préalable, pliée latéralement autour d'un tube ou manchon élastique sous pression, contenant un fluide et façonné pour se conformer à ladite courbure temporaire, la pression dans le tube ou manchon étant adaptée pour être brièvement augmentée en vue de dilater le tube ou manchon et de libérer ainsi les liaisons.

4. Electrode implantable suivant la revendication 1, caractérisée en ce que le moyen de mise sous tension préalable comprend un cordon prévu à l'intérieur ou sur l'élément de support isolant, une traction sur le cordon ou un relâchement de ce dernier ajustant ou commandant de manière continue la courbure de la partie d'électrode à introduire dans la cochlée en vue de fournir la courbure temporaire de cette partie d'électrode.

5. Electrode implantable suivant la revendication 1, caractérisée en ce que le moyen de mise sous tension préalable comprend un tube incurvé présentant une section transversale ovale et contenant un milieu sous pression qui le rend capable de fonctionner comme un tube de Bourdon dans l'ajustement de l'état de courbure en vue de déterminer la courbure temporaire.

0 002 068

FIG. 1

FIG. 4

FIG. 2

FIG. 5

FIG. 6

FIG. 3

FIG. 7